# EUROPEAN PATENT APPLICATION

(11) **EP 1 527 888 A2**
(43) Date of publication of application: **04.05.2005**
(21) Application number: 04014126.9
(22) Date of filing: 16.06.2004
(51) Int. Cl.: B41J 2/21, B41J 2/005, F04B 43/04, C12M 1/00, B01J 19/00

(54) **Printing growth medium for culture and analysis of biological material**

(30) Priority: 30.10.2003 US 699293
(71) Applicant: Hewlett-Packard Development Company, L.P., Houston, Texas 77070 (US)
(72) Inventor: Carpenter, Steven E., Philomath, OR 97370 (US); Dunfield, John Stephen, Corvallis, OR 97330 (US); Ayres, James W., Corvallis, OR 97330 (US)
(74) Representative: Schoppe, Fritz, Dipl.-Ing.

(57) **Abstract**

Sheets of base substrate (12) (e.g., transparency film, glass, or other suitable growth material) are coated with an array of spots (10) of the same or different growth media and/or nutrients. Coating may be accomplished using an ink-jet printer (20), which deposits droplets of a growth medium and/or one or more nutrients on the substrate.

## Description

### FIELD OF THE INVENTION

The present invention relates to the preparation of multiple types of culture media on a substrate for rapid assay of biological material. In particular, it relates to preparation of culture media using ink jet printing mechanisms.

### BACKGROUND OF THE INVENTION

Culture media are traditionally prepared by heat sterilization of agar gel mixed with water and various growth nutrients. The molten agar is poured into culture vessels such as Petri plates, test tubes, or special beakers. Pouring of the media must take place In a sterile or semi-sterile environment in order for contamination not to occur from outside sources. Once the molten agar medium has cooled, it is inoculated with assay microbes in order to determine if the microbes will grow in the nutrient medium upon which they are inoculated.

In the most common methods of performing culture medium assays, Petri plates are used for assaying. In a typical assay of yeasts on different carbohydrate sources, for example, three spots of different carbohydrate sources are placed on a Petri plate. Yeast cells are inoculated near each of the three carbohydrate sources, and the plate is incubated. Yeast growth is determined using a turbidometer or by visual inspection through the thickened growth medium.

A problem with traditional assays such as the above is that they are very time-consuming. Only three spots can generally be used on a typical Petri plate, so a single yeast titer testing 46 carbohydrate sources requires 16 Petri plates, and would typically require a skilled lab worker at least half a day to perform. There remains a need for a faster, more automated method of producing large scale assays.

### SUMMARY OF THE INVENTION

In one aspect, the invention comprises a method of producing a growth medium test sheet, by printing a plurality of growth spots on a surface of a substrate. The spots are printed by depositing drops of growth medium on the substrate.

In another aspect, the invention comprises a growth medium printing system for printing growth spots on a substrate. The system includes at least one print head, each print head connected to a reservoir containing a liquid culture medium or a liquid nutrient and is arranged and constructed to deposit droplets of the liquid culture medium or the liquid nutrient onto selected regions of the substrate to form growth spots. In still another aspect, the invention comprises a method of performing a cell culture, comprising printing a plurality of growth spots onto a substrate by depositing drops of growth medium on the substrate (e.g., via an ink-jet printer), inoculating the printed growth spots by placing cells on the substrate, culturing the cells, and inspecting the growth spots for evidence of cell growth. In yet other aspects, the invention comprises growth medium test sheets comprising a substrate comprising a plurality of growth spots printed thereon, each growth spot comprising a culture medium. The growth spots may be uniform in size and shape, and may have either different or substantially identical compositions.

### BRIEF DESCRIPTION OF THE DRAWING

The invention is described with reference to the several figures of the drawing, in which,
Figure 1 is a transparency sheet that has been printed with an array of growth medium spots according to an embodiment of the invention;
Figure 2 is a printing mechanism for printing growth spots on a substrate according to an embodiment of the invention; and
Figure 3 shows the transparency sheets of Figure 1 after inoculation according to an embodiment of the invention.

### DETAILED DESCRIPTION

"Culturable cells," as that term is used herein, includes all cells capable of being grown in culture, including without limitation yeasts, molds and other fungi, bacteria, algae and other plant cells, and human and other animal cells.

"Growth medium," as that term is used herein, includes agar gels, gelatin, polymers, and all other inert media that may be used for cell cultures.

"Nutrients," as that term is used herein, includes any compound that may be ingested and chemically altered or incorporated into a cell structure by metabolic action, including without limitation carbohydrates and other carbon sources, minerals and mineral salts, proteins and amino acids, lipids and fatty acids, and vitamins.

"Inoculation," as that term is used herein, describes the process of placing a controlled amount of culturable cells on a growth medium.

"Culturing," as that term is used herein, describes a process of holding an inoculated growth medium under conditions that may allow growth of cells. Culturing may include the application of heat and/or moisture, and typically includes isolating the cultured medium from any foreign cells.

"Decoration," as that term is used herein, refers to the process of identifying the presence or quantity of cells via a detectable change in a reagent in contact with the cells (for example, a reagent may be a pH indicator which indicates the pH of a culture via a color change, or a colored stain which preferentially binds to a particular class of cells).

"Elicitor," as that term is used herein, refers to any biochemical substance that may stimulate or inhibit growth of a culture. According to the methods of the invention described below, the effects of various elicitors on culture growth may be studied.

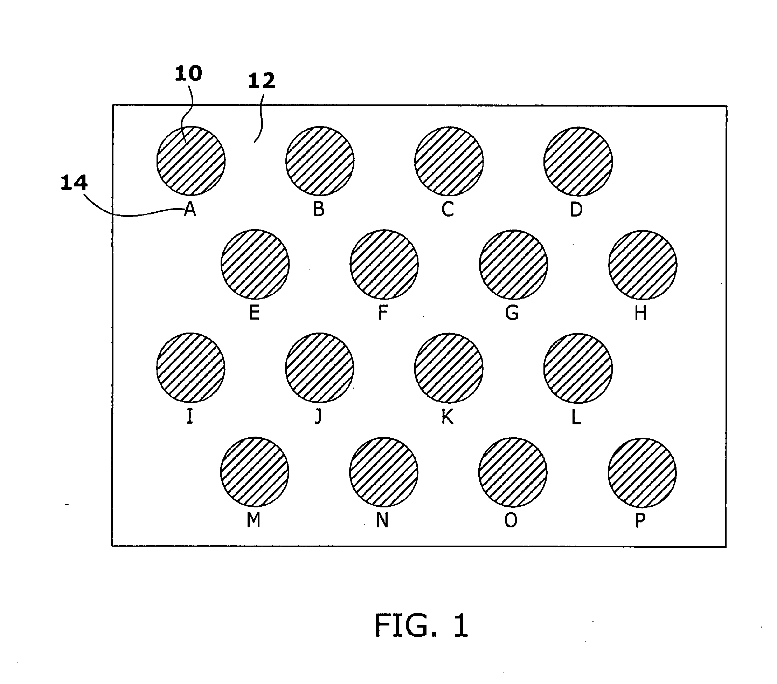

According to the invention, sheets of base substrate (e.g., transparency film, glass, or other suitable growth material, preferably but not necessarily clear) are coated with an array of spots of the same or different growth media and/or nutrients. For example, an array of circles 10 comprising different combinations of growth media and nutrients could be deposited on a transparency sheet 12, as shown in Figure 1.

In the embodiment shown, each of the culture medium growth spots 10 differs in composition from its neighbors. (However, it is within the scope of the invention for some growth spots 10 to comprise the same growth compositions, for example to provide redundancy in measurement or to provide identical starting conditions for a variety of individual cell cultures). The deposited growth spots 10 each consist of a thin layer of one or more growth media components such as agar, and/or nutrients such as a carbohydrate. Preferably, each growth spot 10 has about the same thickness. Further, the growth spots 10 are preferably sufficiently thin to be at least partially transparent, for easier optical detection of cultures, as described below. In some embodiments, the growth spots 10 have labels 14, identifying the contents of each spot.

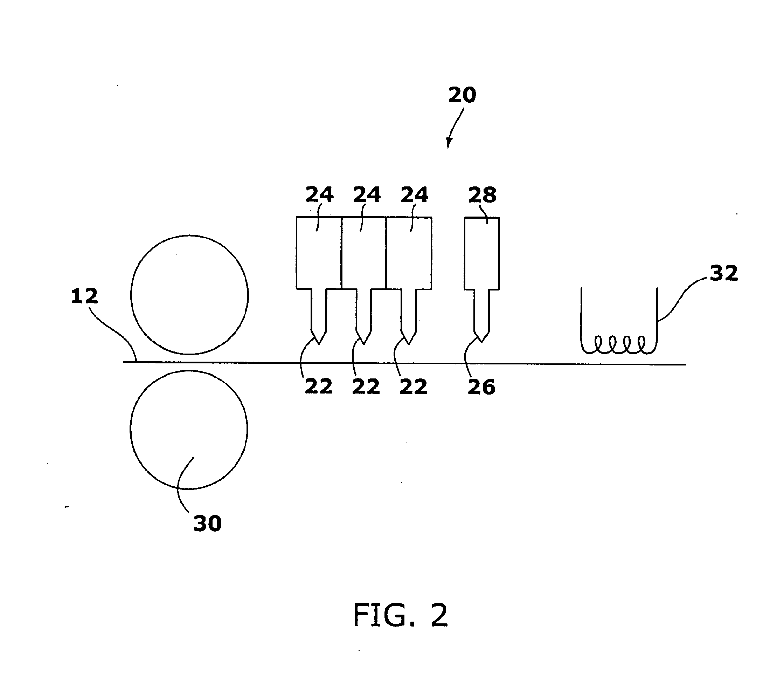

Growth spots 10 are deposited on the substrate 12 by a printing mechanism such as an ink jet printer. An exemplary printer for printing growth spots according to the invention is shown schematically in Figure 2. The printer 20 comprises a plurality of delivery heads 22, each of which is connected to a reservoir 24 containing a different growth medium or nutrient (reservoirs 24 may also comprise additional chemicals and/or biomolecules, such as elicitors, for certain types of assays; for example, it may be desirable to examine the effect of biomolecules such as vitamins on nutrient uptake during culturing). An optional additional print head 26 may be connected to a reservoir 28 comprising a conventional printing ink. The printer further comprises substrate-handling means 30 such as are conventionally known in the art for printing on paper, transparencies, glass, or other print media. A heater 32 or other sterilization means may also optionally be included to guarantee sterility of the substrate 12 and growth medium spots 10.

In use, the printer 20 of Figure 2 prints an array of growth spots 10 on substrate 12, such as that shown in Figure 1, by depositing small drops of growth media and nutrients from reservoirs 24, for example using conventional drop-on-demand ink-jet printing techniques. Other printing techniques known in the art are also included within the scope of the invention. Growth spots 10 may comprise a single growth medium or nutrient, or a mixture of media and/or nutrients produced by overprinting or simultaneous deposition.

In preferred embodiments, the printer 20 also prints a label 14 for each growth spot 10, identifying the contents of the spot. Labels may include text, bar codes, or other identifying indicia, and may be printed in black, color, or multiple colors.

Before or after the growth spots are printed on the substrate 12, the printer may optionally include sterilization means, such as a heater 32, an ultraviolet lamp (not shown), or other known mechanisms for sterilizing culture media before inoculation.

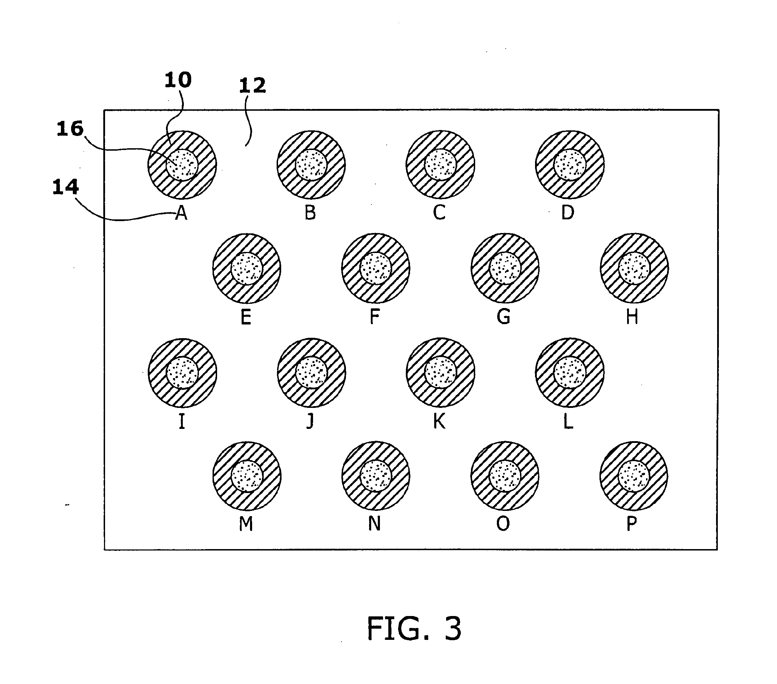

After sterilization (if any), test organisms such as yeast, mold, bacteria, or other cells are inoculated onto the substrate 12. Inoculation may be performed by printing a solution of the organism in the same manner as printing the growth spots 10, or may be manually performed on a preprinted sheet 12. If printing of the organism is done in the same apparatus as the growth spots, then the amount and placement of organism at each growth spot 10 may be precisely controlled. However, in this case, it is necessary to have all appropriate growth media and nutrient reservoirs 24 at the location where experiments will be carried out, so that printing can occur simultaneously. Alternatively, preprinted sheets of the various culture media spots can be prepared and stored under sterile conditions until use. These sheets can then be inoculated either by hand or using a printer, whenever a test of an organism is to be performed. Figure 3 shows the test sheet of Figure 1 after inoculation. Inoculation spots 16 have been printed at the center of each growth spot 10.

After inoculation, the substrate 12 is cultured using conventional techniques to allow growth of the test organism (for example, by holding the substrate under appropriate temperature and humidity conditions for a time sufficient for significant cell division to occur). The substrate 12 is then inspected to determine which growth media promote growth of the test organism. For some embodiments, positive growth can be determined by placing the sheet into a scanner or other analyzer to measure turbidity via either transmitted or reflected light. In addition, particularly for transparent substrates, growth may be visible to the naked eye.

It is also within the scope of the invention to decorate the growth spots in order to enhance detection of growth in culture. Decoration reagents that do not adversely affect growth of the test organism may be applied before growth occurs, while reagents having undesirable toxic effects should be applied after culturing. In either case, decoration reagents may be applied via printing, in the same manner as the growth media and nutrients, or they may be applied by hand. Nontoxic reagents may be printed at the same time as the growth spots 10. When applying reagents after culturing, the substrate 12 may be fed through a printer a second time to place the reagents. After decoration, growth is observed using a scanner or other analyzer as described above. Figure 4 shows the test sheet of Figure 3 after culturing and decoration; different growth spots 10 have different darknesses corresponding to the degree of growth of the cell cultures. (Using other decorating reagents, the hue of the growth spots may also reflect the degree of cell growth).

Electronic metrology of the growth spots 10 is enhanced when they are printed according to the invention, because of the consistency of printing as compared with conventional manual methods of deposition of growth media. In addition, the ability to print labels on test sheets at the same time as the growth spots helps prevent confusion in interpreting assay results.

In addition to conventional static analyses of growth, the methods of the invention may also be employed to obtain more dynamic information. Automated analysis of cell growth may be performed in real time or time lapse in order to obtain dynamic cell growth information. For example, "movies" of cell growth progression may be obtained by scanning the sheet at regular intervals (which may be short enough to effectively provide real-time information). Alternatively, non-image data characterizing different growth spots (e.g., turbidity, size, and/or color) may be recorded at intervals. These data may be compared, for example, to determine the effects of different media and nutrients on the rate of cell growth, or on comparative growth characteristics of different organisms.

Dynamic methods may also be used to capture the effects of one or more elicitors on organism(s). For example, different elicitors may be added to different growth spots to determine their effects on growth rate. Both growth rate and growth morphology may be measured as a function of time in order to characterize the interaction of cells and elicitors. When characterizing cell morphology, high magnification is preferably used so that the characteristics of individual cells may be observed. Transient behavior of cells may also be examined by use of high-speed image capture immediately after addition of an elicitor.

One embodiment of the invention is a fully automated cell analysis workstation. The workstation comprises a printer, used as described above to deposit growth media, cells, and optionally nutrients, elicitors, decorating reagents, and/or other biomolecules, and a suitable culture area for holding a sheet of growth cells under appropriate culture conditions. The workstation further comprises a scanner, camera, or other imaging system for measuring culture growth, either a single time or dynamically. The workstation preferably also comprises a microprocessor, suitably programmed for conducting image analysis, pattern recognition, transient analysis, combinatorial statistics, or other computational operations on the measured cultures. A user may simply program the workstation for a desired experiment, providing appropriate cells and reagents, and the automated system will carry out the experiment and provide results in the desired format, including providing images of the cultures and analysis of their growth patterns.

### Examples

In a specific example, the invention is used to assay for the presence of beta-galactosidase activity in plasmid-transformed E. coli bacteria strains with genomes that do not produce beta-galactosidase. After plasmid transformation of the genome, some individual bacteria will contain the ability tb produce beta-galactosidase. Growth media are printed according to the invention using a mixture of agar and X-gal, which turns blue in the presence of beta-galactosidase. Small quantities of transformed bacteria are then overprinted in the growth medium spots, and the sheet is cultured to allow growth. Culture spots that turn blue contain the plasmid-transformed E. coli colonies that contain the genome for producing beta-galactosidase. These spots may be automatically detected using conventional scanning technology.

In another example, different sets of growth media can be printed on the same sheet, each printed area consisting of a single growth medium, and each growth medium differing from the others by different carbohydrate composition. Yeast or bacteria cells can then be printed on these spots. Growth on the printed area indicates positive metabolism of the carbohydrate. Carbohydrate tests of this type are very useful in differentiating yeast species.

An elicitor can be added to colonies that have been grown from a printed array. The array can be exposed to biochemical toxins that cause changes in cellular activity, such as cells that change color when exposed to the toxin.

Other embodiments of the invention will be apparent to those skilled in the art from a consideration of the specification or practice of the invention disclosed herein. It is intended that the specification and examples be considered as exemplary only, with the true scope and spirit of the invention being indicated by the following claims.

## Claims

1. A method of producing a growth medium test sheet, comprising:
printing a plurality of growth spots on a surface of a substrate by depositing drops of growth medium on the substrate.

2. The method of claim 1, wherein printing comprises depositing a nutrient in at least one of the plurality of growth spots.

3. The method of claim 1 or 2, wherein the plurality of growth spots comprise individual spots composed of all or a portion of the possible combinations of
(a) a first group of one or more growth media; and
(b) a second group of one or more nutrients.

4. The method of any of claims 1-3, wherein printing comprises printing with an ink-jet printer.

5. A method of performing a cell culture, comprising:
printing a plurality of growth spots onto a substrate by the methods of any of claims 1-4;
inoculating the printed growth spots by placing cells on the substrate;
culturing the cells on the substrate; and
inspecting the growth spots for evidence of cell growth.

6. A growth medium printing system for printing growth spots on a substrate, comprising:
at least one print head, wherein each print head is connected to a reservoir containing a liquid culture medium or a liquid nutrient, and wherein each print head is arranged and constructed to deposit droplets of the liquid culture medium or the liquid nutrient onto selected regidns of the substrate to form growth spots.

7. The growth medium printing system of claim 6, wherein the system comprises a plurality of print heads, wherein each print heads connected reservoir contains a different composition of liquid culture medium or liquid nutrient.

8. The growth medium printing system of claim 6 or 7, further comprising a sterilizer that sterilizes the printed growth spots.

9. The growth medium printing system of any of claims 6-8, further comprising
a print head connected to a cell reservoir and arranged and constructed to inoculate cells onto the substrate; or
a print head connected to a decorating reagent reservoir and arranged and constructed to print reagent onto growth spots on the substrate.

10. An automated cell analysis workstation, comprising:
a printer comprising at least one print head, wherein each print head is connected to a reservoir containing a liquid culture medium, a liquid nutrient, or a culturable organism, and wherein each print head is arranged and constructed to deposit droplets of the liquid culture medium, liquid nutrient, or culturable organism onto selected regions of a substrate to form growth spots;
a culturing chamber comprising means for maintaining a selected temperature in a sterile environment;
an imaging device capable of detecting growth in culture of the culturable organism;
one or more microprocessors suitably programmed to control the printer and culturing chamber and to record images obtained by the imaging device or data derived from images obtained by the imaging device; and
an input/output system allowing a user to select printing parameters and culturing conditions and to view or save the images or data derived from images.
